# EUROPEAN PATENT APPLICATION

(11) **EP 1 935 978 A1**
(43) Date of publication of application: **25.06.2008**
(21) Application number: 06798170.4
(22) Date of filing: 14.09.2006
(51) Int. Cl.: C12N 15/09, A61K 45/00, A61K 48/00, A61K 49/00, A61P 35/00, A61P 37/02, C07K 14/245, C07K 19/00, C12N 1/15, C12N 1/19, C12N 1/21, C12N 5/10, C12Q 1/02

(54) **METHOD OF CONTROLLING DECOMPOSITION OF PROTEIN BY TETRACYCLINE ANTIBIOTIC**

(30) Priority: 15.09.2005 JP 2005269074
(71) Applicant: Japan Science and Technology Agency, Kawaguchi-shi, Saitama 332-0012 (JP)
(72) Inventor: MIWA, Yoshihiro, Tsukuba-shi, Ibaraki 305-0031 (JP)
(74) Representative: Gillard, Richard Edward
(86) International application number: PCT/JP2006/318673
(87) International publication number: WO 2007/032555

(57) **Abstract**

The present invention provides a fusion protein comprising a variant protein of a protein binding to an antibiotic and a target protein having fused thereto, wherein the variant protein is degraded when the antibiotic is not bound but stabilized when the antibiotic is bound in a cell, and the fusion protein is degraded when the antibiotic is not bound but stabilized when the antibiotic is bound in a cell.

## Description

### TECHNICAL FIELD

The present invention relates to a method of controlling the degradation of a protein by antibiotics capable of being introduced into cells, and a fusion protein used therefor. Specifically, the present invention relates to a method of controlling the degradation of a protein by tetracycline antibiotics.

### BACKGROUND ART

In December 2003, the group of Crabtree, et al. from Stanford University reported on a system for controlling proteolytic degradation by a rapamycin derivative as an immunosuppressor in combination with a mutant of FRAP (FKBP12-rapamycin-associated protein) as its binding protein (Stankunas, K. et al., Molecular Cell, Vol. 12, 11815-1824, December, 2003).

The system for controlling the degradation of a protein in accordance with the conventional art consists of three elements of the FRAP mutant, rapamycin derivative and FKBP12 protein originally present in cells. An 89 amino acid domain FRB, which is the minimal region of FRAP required for FKBP12-rapamycin binding, is used as the FRAP mutant. FRB destabilizes glycogen synthase kinase-3β (GSK-3β) when FRB is fused to GSK-3β, but in the presence of the rapamycin derivative, the structure of GSK-3βFRB fusion protein is stabilized by binding to the FKBP12 protein which is ubiquitously present, and both the protein level and activity thereof can be restored.

### DISCLOSURE OF INVENTION

In Stankunas, K. et al. (2003), the FKBP12 protein originally present in cells is also used, in addition to the FRB protein which is expressed in cells following gene transduction, to stop the degradation of GSK-3β. Thus, normal proteins are co-present in cells, and the control of the degradation is only possible when the fusion protein binds to another protein. It is therefore absolutely necessary to carry out any experiments, while considering effects on cells. Furthermore, the rapamycin derivatives used in Stankunas, K. et al. (2003) have not been used clinically, and any unwanted actions such as immunosuppression, etc. may occur and it is still unclear how such actions would affect organisms. Waste materials after experimentation also require careful handling.

Accordingly, an alternative system or easy-to-use system for controlling the protein level in living cells using an artificial degradation control mechanism has been desired.

The present inventors has prepared a variant tetracycline repressor protein of which the degradation can be repressed by tetracycline antibiotics and expressed in cells as a fusion protein with a target protein, the level of which is to be controlled in cells. The inventors have found that the degree of degradation of the target protein can be controlled artificially in the presence or absence of tetracycline antibiotics or by varying the amount of these antibiotics, and have accomplished the present invention.

That is, the present invention provides a fusion protein, a polynucleotide encoding the fusion protein, an expression vector comprising the polynucleotide, a composition comprising the fusion protein, the polynucleotide or the expression vector, a kit and method for controlling the degradation of a target protein, which are described below.
(1) A fusion protein comprising a variant protein of a protein binding to an antibiotic and a target protein having fused thereto, wherein:
   said variant protein is degraded when the variant protein is not bound to said antibiotic but stabilized when the variant protein is bound to said antibiotic in a cell; and,
   said fusion protein is degraded when the fusion protein is not bound to said antibiotic but stabilized when the fusion protein is bound to said antibiotic in a cell.
(2) The fusion protein according to item (1), wherein said antibiotic is tetracycline antibiotic, the protein bound to said antibiotic is a repressor protein of said antibiotic.
(3) The fusion protein according to item (2), comprising a variant TetR protein and a target protein having fused thereto.
(4) The fusion protein according to item (3), wherein said variant TetR protein has an amino acid sequence in which at least one amino acid residue is substituted in the amino acid sequence of wild-type TetR protein.
(5) The fusion protein according to item (4), wherein at least two amino acid residues of aspartic acid at position 95, leucine at position 101 and glycine at 102 are substituted in the amino acid sequence of wild-type TetR protein.
(6) The fusion protein according to any one of items (1) through (5), wherein said target protein is a fluorescent protein or a luminescent protein.
(7) The fusion protein according to any one of items (1) through (5), wherein said target protein is a therapeutic protein.
(8) The fusion protein according to any one of items (1) through (5), wherein said target protein is a protein provided for functional analysis.
(9) The fusion protein according to item (6), wherein said fluorescent protein or luminescent protein is any one of a green fluorescent protein and a luciferase.
(9a) The fusion protein according to item (4), wherein said variant TetR protein has an amino acid sequence containing at least two of the mutations that asparagine is substituted for aspartic acid at position 95, serine for leucine at position 101 and aspartic acid for glycine at 102 in the amino acid sequence of wild-type TetR protein, and said target protein is a fluorescent protein or a luminescent protein.
(9b) The fusion protein according to item (9a), wherein said variant TetR protein contains an additional mutation that glutamine is substituted for arginine at position 28 in the amino acid sequence of said wild-type TetR protein.
(10) A polynucleotide encoding the fusion protein according to items (1) through (9).
(10a) A composition comprising the polynucleotide according to item (10).
(11) An expression vector comprising a polynucleotide encoding a fusion protein according to items (1) through (9).
(12) A host cell or host organism, which is transfected by the expression vector according to item (11).
(13) A composition for intracellular or *in vivo* imaging, comprising the fusion protein according to item (6), (9), (9a) or (9b).
(14) A composition for intracellular or *in vivo* imaging, comprising an expression vector comprising a polynucleotide encoding the fusion protein according to item (6), (9), (9a) or (9b).
(15) A composition for treatment, comprising the fusion protein according to item (7).
(16) A composition for treatment, comprising an expression vector comprising a polynucleotide encoding the fusion protein according to item (7).
(17) A composition for analysis of protein functions, comprising the fusion protein according to item (8).
(18) A composition for analysis of protein functions, comprising an expression vector comprising a polynucleotide encoding the fusion protein according to item (8).
(19) The composition according to any one of items (13) through (18), which is used in combination with a tetracycline antibiotic.
(19a) The composition according to any one of items (13) through (18), further comprising a tetracycline antibiotic.
(20) The composition according to item (19) or (19a), wherein said tetracycline antibiotic is tetracycline or its derivative selected from doxycycline, oxytetracycline and chlorotetracycline, or anhydrotetracycline.
(21) A kit comprising the fusion protein according to any one of items (1) through (9), the polynucleotide according to item (10), the expression vector according to item (11) or the composition according to items (13) through (20).
(2 a) The kit according to item (21), further comprising a tetracycline antibiotic.
(22) A method for controlling the degradation of a protein by an antibiotic capable of being introduced into a cell, which comprises any one of the steps (A) and (B):
(A) the step of expressing a polynucleotide encoding a fusion protein comprising a variant protein of a protein binding to said antibiotic and a target protein having fused thereto in a cell or *in vivo* in the presence or absence of said antibiotic, and,
(B) the step of using a fusion protein comprising a variant protein of a protein binding to said antibiotic and a target protein having fused thereto in a cell or *in vivo* in the presence or absence of said antibiotic, wherein:
   said variant protein is degraded when the variant protein is not bound to said antibiotic but stabilized when bound to said antibiotic in a cell; and,
   said fusion protein is degraded when the fusion protein is not bound to said antibiotic but stabilized when the fusion protein is bound to said antibiotic in a cell.
(23) The method according to item (22), wherein said antibiotic is a tetracycline antibiotic, the protein bound to said antibiotic is a repressor protein of said antibiotic.
(24) A method for controlling the degradation of a protein by a tetracycline antibiotic, which comprises any one of the steps (A) and (B):
   (A) the step of expressing a polynucleotide encoding a fusion protein comprising a variant TetR protein and a target protein having fused thereto in a cell or *in vivo* in the presence or absence of the tetracycline antibiotic, and,
   (B) the step of using a fusion protein comprising a variant TetR protein and a target protein having fused thereto in a cell or *in vivo* in the presence or absence of the tetracycline antibiotic.
(25) The method according to item (23) or (24), wherein the degradation of said target protein is controlled by regulating the level of said tetracycline antibiotic.
(26) The method according to item (24) or (25), wherein said variant TetR protein has an amino acid sequence in which at least two amino acid recidues of aspartic acid at position 95, leucine at position 101 and glycine at 102 are substituted in the amino acid sequence of wild-type TetR protein.
(27) The method according to any one of items (22) through (26), wherein said target protein is a therapeutic protein
(28) The method according to any one of items (22) through (26), wherein said target protein is a protein for functional analysis.
(29) The method according to any one of items (22) through (26), wherein said target protein is a fluorescent protein or a luminescent protein.
(29a) The method according to item (29), which is for use in intracellular or *in vivo* imaging.
(30) The method according to any one of items (23) through (29a), wherein said tetracycline antibiotic is tetracycline or its derivative selected from doxycycline, oxytetracycline or chlorotetracycline, or anhydrotetracycline.

According to the present invention, analysis of the target protein for its function, pharmacokinetic analysis by imaging, treatment of diseases with minimized side effects, etc. can be performed by using the simple molecular system.

The tetracycline antibiotic used in the present invention has the benefits that the antibiotic is very inexpensive, and excellent in its absorption and permeability when it is used in animal, and its high safety as compared with rapamycin used in Stankunas, K., et al. (2003). The tetracycline antibiotics used in the present invention are advantageous in that there are very many experimental studies on administration to mice, these antibiotics are drugs already used widely in the clinical area and their safety has been established.

In the method of the present invention for controlling the degradation of a protein, proteins (e.g., proteins originally present in cells) other than the fusion protein, which is delivered to cells from the outside, are not used. For this reason, any undesired effects on cells can be avoided.

### BRIEF DESCRIPTION OF THE DRAWINGS

FIG. 1 is (A) a schematic diagram of cDNA encoding TetR-EGFP; and (B) - (D) are graphs showing the results obtained when the stability of wild-type TetR-EGFP and variant TetR-EGFP was assessed using a flow cytometer.
FIG. 2 is photographs showing changes in fluorescence when the cells where the variant TetR-EGFP was expressed were incubated in the presence of doxycycline. (A): no substitution of arginine at position 28 is present, (B): substitution of arginine at position 28 with glutamine is present.
FIG 3 is a graph showing the results of analysis of intensity of fluorescence in the cells where the variant TetR-EGFP was expressed, by adding doxycycline in various concentrations.
FIG. 4A is a graph showing changes in intensity of fluorescence in the cells expressing the variant TetR-EGFP after addition of doxycycline, as compared to the cells expressing EGFP only. FIG 4B is a graph showing changes in intensity of fluorescence in the cells expressing the variant TetR-EGFP after removal of doxycycline, as compared to the cells expressing only EGFP.
FIG. 5 is photographs showing the results of observation of fluorescence in mice, in which the vector incorporated with the genes for variant TetR-EGFP and DsRed was injected and expressed, with passage of time, using an inverted microscope.
FIG 6 is a graph showing the intensity of fluorescence with passage of time in the experiments shown in FIG 5.
FIG. 7 is a photograph showing the results obtained when the variant TetR-EGFP-encoding mRNA was injected into fertilized zebrafish eggs and the fluorescence was observed on an inversed microscope in the presence or absence of doxycycline. (A): intact cells in the absence of doxycycline, (B): intact cells in which doxycycline was added, (C): cells in which the variant TetR-EGFP-encoding mRNA was introduced and doxycycline was added.
FIG. 8 is a graph showing the results obtained when the viable cell count was compared in cells, in the presence or absence of doxycycline, between the method of controlling the transcription of toxin gene by tetracycline alone and the method of the present invention for controlling the degradation of proteins.

### BEST MODE FOR CARRYING OUT THE INVENTION

The present invention provides a variant protein in which a point mutation is introduced into *Escherichia coli*-derived tetracycline repressor protein (TetR protein). More specifically, the present invention provides the fusion protein comprising a variant protein in which a point mutation is introduced into TetR protein and the target protein having fused thereto. The fusion protein of the present invention has acquired the property that, in the living animal cells, the fusion protein is unstable and undergoes rapid degradation in itself but is stabilized by binding to the tetracycline antibiotic so as to avoid the degradation. The present invention can be used for the purposes described in 1) to 3) below.
1) The *Escherichia coli* tetracycline repressor (TetR) protein is stable in human cells. However, the variant, into which mutations (e.g., two or more) are introduced, is rapidly degraded in the absence of the tetracycline antibiotic (sometimes abbreviated as "Tet" throughout the specification), but the degradation can be avoided by adding Tet and the variant is accumulated in cells. Then, when the fusion protein of the variant TetR protein and a target protein, function of which is to be subjected to analysis, is expressed in cells, the level of the target protein in cells can be controlled by the amount of Tet added. This enables empirical analysis of the effects that the target protein would impose on cells or individual organisms.
2) Where a foreign gene is introduced into a patient for gene therapy to improve the conditions by the action of a protein, i.e., the gene product, there is a risk that the protein might act as an antigen to cause unexpected adverse effects. Now, when a fusion gene of the target gene for treatment and the variant TetR protein is introduced into a patient, the amount of the gene product, i.e., the fusion protein, can be controlled by Tet and Tet can be administered to a patient depending on the condition of the patient, which enables treatment of the condition while avoiding adverse effects.
3) Where the variant TetR protein is expressed in cells in the form of the fusion protein with a detectable protein such as a fluorescent protein or a luminescent protein, the fluorescence level or luminescence level changes depending on the amount of Tet so that imaging is enabled by detecting the amount of Tet in living cells or individual organisms.

### 1. A fusion protein of the present invention

In one aspect, the present invention therefore provides a fusion protein comprising a variant protein of a protein binding to an antibiotic and a target protein having fused thereto. Here, the variant protein is degraded when the variant protein is not bound to the antibiotic but stabilized when the variant protein is bound to the antibiotic in a cell; and, the fusion protein is degraded when the fusion protein is not bound to the antibiotic but stabilized when the fusion protein is bound to the antibiotic in a cell.

As used herein, the "variant protein of a protein binding to an antibiotic" refers to a variant of the protein comprising the amino acid sequence wherein at least one amino acid residue is substituted, deleted, added or inserted in the amino acid sequence of the protein binding to the antibiotic, and is destabilized and degraded by a protease in the absence of the antibiotic, but is stabilized to avoid the degradation when bound to the antibiotic.

Examples of the "antibiotic" include tetracycline antibiotics, penicillin antibiotics, chloramphenicol antibiotics, aminoglycoside antibiotics, etc. Examples of the "protein" binding to such antibiotics include a repressor protein of the antibiotic, β-lactamase, chloramphenicol acetyltransferase, aminoglycoside 3'-phosphotransferase, etc. In a preferred embodiment of the present invention, the antibiotic described above is a tetracycline antibiotic, and the protein binding to the antibiotic described above is a repressor protein of the antibiotic.

Preferably, the repressor protein is the TetR protein, and the variant protein is the variant TetR protein. Therefore, the present invention provides the fusion protein comprising the variant TetR protein and a target protein having fused thereto.

As used herein, the "TetR protein" or "wild-type TetR protein" refers to the *Escherichia coli*-derived tetracycline repressor protein (NCBI protein database Accession No: NP_941292 (SEQ ID NO: 2); CDS: NC_005211 (SEQ ID NO: 1)).

As used herein, the "variant TetR protein" refers to a variant of the TetR protein comprising the amino acid sequence wherein at least one amino acid residue is substituted, deleted, added or inserted in the amino acid sequence of the TetR protein, and is destabilized and degraded by a protease in the absence of the tetracycline antibiotic, but is stabilized to avoid the degradation by binding to the tetracycline antibiotic.

Preferably, the variant TetR protein described above has the amino acid sequence that at least two amino acid residues are substituted in the amino acid sequence of the wild-type TetR protein. More preferably, the substitution of the amino acid residues is present in at least two positions of aspartic acid at position 95, leucine at position 101 and glycine at 102 in the amino acid sequence of the wild-type TetR protein. Most preferably, the variant TetR protein described above has the amino acid sequence having either at least two or all of the three mutations in which asparagine is substituted for aspartic acid at position 95, serine for leucine at position 101 and aspartic acid for glycine at position 102 in the amino acid sequence of the wild-type TetR protein. The mode of mutations in the amino acid sequence of variant TetR protein may include not only the substitution of amino acid residues from the wild-type TetR protein but also deletion, addition and/or insertion of one or more amino acid residues. The position of the amino acids having such mutations is not limited to those given by way of the examples described above.

In the fusion protein of the present invention, the "target protein" is meant to refer to a protein providing industrially useful effects through controlling the degradation (or stability or activity) of the protein utilizing the variant TetR protein and Tet, such as (1) a fluorescent protein or a luminescent protein; (2) a therapeutic protein; or (3) a protein for subjecting to analysis of its function, etc. Where the target protein is a known protein, the nucleotide sequence encoding the protein is usually available from various publicly accessible sequence databases (e.g., the GenBank database). Where an amino acid sequence of the target protein or a nucleotide receptor sequence encoding the same is unknown, the amino acid sequence of the protein and the nucleotide sequence encoding the same can be determined by sequencing methods well-known to those skilled in the art (cf., e.g., Sambrook & Russell, Molecular Cloning: A Laboratory Manual, Third Edition, 2001, Cold Spring Harbor Laboratory Press, Cold Spring Harbor, New York, etc.).

The fusion protein of the present invention can be prepared by conventional methods in the art. Briefly, the fusion protein can be prepared by ligating cDNA encoding the variant TetR protein with cDNA encoding the target protein to construct the DNA encoding the fusion protein of the target protein and the variant TetR protein, inserting this DNA into, for example, an expression vector for eukaryotes, and introducing the expression vector into a eukaryote to express the fusion protein (cf., e.g., Sambrook & Russell *supra*).

Examples of the "fluorescent protein" used in the embodiments of the present invention include a green fluorescent protein (GFP), an enhanced green fluorescent protein (EGFP), a cyan fluorescent protein (CFP)), an enhanced cyan fluorescent protein (ECFP), a yellow fluorescent protein (YFP), an enhanced yellow fluorescent protein (EYFP), a red fluorescent protein DsRed and its variants (DsRed2, DsRed-express, timer and mRFP1, and their variants, etc.), AmCyan, ZsGreen, ZsYellow, AsRed, HcRed, KusabiraOrange, Kaede, AzamiGreen, and the like.

Examples of the "luminescent protein" used in the embodiments of the present invention include firefly luciferase, Renilla luciferase, jellyfish aequorin, etc. These fluorescent proteins or luminescent proteins are commercially available from suppliers well-known to those skilled in the art (e.g., Clontech, Promega, etc.).

As used herein, the "therapeutic protein" refers to a protein which is effective for the prevention and/or treatment of diseases and is exemplified by, for example, cytokines activating immune-mediating cells (e.g., human interleukin 2, human granulocyte-macrophage-colony-stimulating factor, human macrophage-colony-stimulating factor, human interleukin 12, etc.), and the like. Lysins, toxins such as diphtheria toxin, etc., or herpes virus thymidine kinase can also be used in combination with anti-viral agent ganciclovir to kill cancer cells directly. Furthermore, antibodies or the like can also be used. Turning to the fusion protein with, e.g., an antibody, a cDNA encoding the variant TetR protein is ligated with a cDNA encoding an antibody or antibody fragment to construct the DNA encoding the fusion protein of the antibody and the variant TetR protein, this DNA is inserted into, e.g., an expression vector for eukaryotes, and the expression vector is introduced into eukaryotes, whereby the fusion protein can be expressed. Alternatively, in order to deliver a site-specific therapeutic protein to a particular antigen in biological tissues, a DNA encoding an antibody to the antigen, the therapeutic protein and the variant TetR protein is constructed, this DNA is inserted into, e.g., an expression vector for eukaryotes, the expression vector is introduced into eukaryotes, and thus the fusion protein can be expressed. Alternatively, such a fusion protein may be prepared *ex vivo* and then introduced into the living body.

The "protein for subjecting to analysis of its function" used in another embodiment of the present invention includes, for example, a protein kinase, a transcription factor, etc. Examples of the protein kinase are MAPK family kinase, PKC family kinase, PKA family kinase, Src family kinase, Jak family kinase, Abl family kinase, IKK family kinase, etc. Examples of the transcription factor are, RUNX family, STAT family, nuclear receptor, leucine zipper family, NF-κB family, etc.

The fusion protein of the present invention described above is unstable in the absence of the tetracycline antibiotic but stabilized when it is bound to the tetracycline antibiotic. Accordingly, the fusion protein of the present invention enables to control the degradation of the target protein by the concentration of the tetracycline antibiotic. Thus, the fusion protein of the present invention can be used for *in vivo* imaging using the fluorescent or luminescent protein, control of effects of the therapeutic protein, analysis of the fusion protein of its function, and the like.

The "tetracycline antibiotic (Tet)" used in the present invention is not particularly limited, so long as it is bound to the variant TetR protein of the present invention to stabilize its structure and suppress the degradation by a protease, and therefore exemplified by tetracycline and its derivatives such as doxycycline, oxytetracycline, chlorotetracycline, and anhydrotetracycline.

### 2. Polynucleotide and expression vector of the invention and host transfected using the same

In another aspect, the present invention provides a polynucleotide encoding the fusion protein of the present invention. The present invention further provides an expression vector comprising the polynucleotide encoding the fusion protein of the present invention. Preferably, the expression vector of the present invention comprises an expression cassette comprising the elements (a) to (c) below:
(a) a promoter enabling transcription in a host cell;
(b) a polynucleotide bound to the promoter and encoding the fusion protein of the present invention; and,
(c) a signal that functions in the host cell in association with the transcription termination and polyadenylation of an RNA molecule.

The promoter and the transcription termination signal (terminator) are used in an appropriate combination to increase the efficiency of gene expression, depending upon a host into which the expression cassette described above is to be introduced. One skilled in the art can suitably choose such an appropriate combination. A non-limiting example of such an expression vector includes the expression vector pEB6CAG used in EXAMPLES of the present invention, which is replicated and maintained stably in human cells. This vector contains CAG promoter as a promoter, the variant TetR-EGFP as the fusion protein and SV40polyA sequence as a transcription termination signal sequence.

The expression vector of the present invention may also contain other elements in addition to the expression cassette described above. Non-limiting examples of such additional elements include an IRES sequence as used in EXAMPLES of the specification, which is inserted between the variant TetR-EGFP and SV40polyA, and a cDNA capable of expressing such a fluorescent protein as the tandem dimer of DsRed downstream of the IRES sequence.

Additional examples of the expression unit or expression vector which can be used to express the fusion protein of the present invention in cells or *in vivo* include expression units found in plasmid pcDNA3 (Invitrogen), plasmid AH5, pRC/CMV (Invitrogen), pCAGGS, pCXN2, pME18S, pEF-BOS, etc. The introduction of genes into expression units and/or vectors can be accomplished using genetic engineering techniques, as described in manuals including Molecular Cloning & Current Protocols in Molecular Biology (Sambrook, J., et al., Molecular Cloning, Cold Spring Harbor Press (1989); Ausbel, F.M., et al., Current Protocols in Molecular Biology, Green Publishing Associates and Wiley-Interscience (1989), etc. A resulting expressible polynucleotide can be introduced into cells of a subject (e.g., a human subject) by any method capable of placing the polynucleotide into cells in an expressible form (for example, as naked plasmid or other DNA, as part of a viral vector, or in targeted liposomes. Methods for gene transduction include direct injection into tissues or affected areas (e.g., tumors), liposomal transfection (Fraley, et al., Nature, 370: 111-117 (1980)), receptor-mediated endocytosis (Zatloukal, et al., Ann. N.Y Acad. Sci., 660: 136-153 (1992)), and particle bombardment-mediated gene transfer (Eisenbraun, et al., DNA & Cell. Biol. 12:791-797 (1993))

In one aspect, the present invention further provides host cells or host organisms, into which the polynucleotide is introduced or which are transfected by the expression vector described above. Non-limiting examples of such host cells or host organisms include vertebrates and their cells; for example, fish, amphibians, reptiles, fowls, mammals, etc. or their cells can be used. In addition, insects and their cells can also be used. Examples of mammals are humans, mice, rats, rabbits, sheep, swine, porcine, horses, fowls, cats, dogs, monkeys, chimpanzees, etc. More specific examples include, but are not limited to, human cells, mice, fertilized eggs of zebrafish, etc., used in EXAMPLES of the present invention.

### 3. Composition comprising the Fusion protein of the invention or the polynucleotide encoding the fusion protein

In a further aspect, the present invention provides a composition comprising the fusion protein of the present invention, a composition comprising the polynucleotide encoding the fusion protein of the present invention, and a composition comprising the expression vector comprising the polynucleotide encoding the fusion protein of the present invention. These compositions of the present invention are used in combination with the antibiotic binding to the fusion protein described above. For example, where the fusion protein described above contains a variant of the tetracycline antibiotic repressor protein, the composition of the present invention is used in combination with the tetracycline antibiotic (tetracycline and its derivatives such as doxycycline, oxytetracycline, chlorotetracycline, and anhydrotetracycycline).

In one embodiment, the composition of the present invention comprises a fusion protein of a fluorescent protein or a luminescent protein and a variant protein of a protein binding to the antibiotic, or comprises an expression vector comprising the polynucleotide encoding such a fusion protein. A preferred example of the variant protein is the variant TetR protein. Preferred examples of the fluorescent protein or the luminescent protein include the same as those already given for describing the fusion protein of the present invention. Preferred examples of the variant TetR protein are the same as those already given for describing the fusion protein of the present invention. In order to prevent predominant accumulation of the fluorescence in the nucleus of a cell, preferably the variant TetR protein described above may further have substitution of glutamine for arginine in the amino acid residue at position 28. In the fusion protein of the present invention, its degradation can be controlled by the concentration of Tet. Therefore, the composition of the present invention can be used for detecting the amount of the tetracycline antibiotic in a cell or *in vivo* by imaging and can be used for monitoring pharmacokinetics.

In another embodiment, the composition of the present invention comprises a fusion protein of a therapeutic protein and a variant protein of a protein binding to the antibiotic, or comprises an expression vector comprising a polynucleotide encoding such a fusion protein. A preferred example of the variant protein is a variant TetR protein. Preferred examples of the therapeutic protein include the same as those already given for describing the fusion protein of the present invention. Preferred examples of the variant TetR protein are the same as those already given for describing the fusion protein of the present invention. Where a foreign gene is introduced into a patient for gene therapy to improve its conditions by the action of a protein, i.e., the gene product, there is a risk that the protein might act as an antigen to cause unexpected adverse effects. When a fusion gene of the gene encoding the therapeutic protein and variant TetR is introduced, the expression level of the fusion protein, i.e., the gene product, can be controlled by Tet and Tet can be administered to a patient depending on the conditions, which enables treatment while avoiding adverse effects.

In a further embodiment, the composition of the present invention comprises a fusion protein of a protein for subjecting to analysis of its function and a variant protein of a protein binding to the antibiotic, or comprises an expression vector comprising a polynucleotide encoding such a fusion protein. A preferred example of the variant protein is a variant TetR protein. Preferred examples of the protein for subjecting to analysis of its function include the same as those already given for describing the fusion protein of the present invention. Preferred examples of the variant TetR protein are the same as those already given for describing the fusion protein of the present invention. Using the composition of the present invention, a fusion protein of, e.g., the variant TetR and the target protein, which function is to be subjected to analysis, is expressed in a cell, and the level of the target protein in the cell can be controlled by the amount of Tet added. By doing so, the effect that the target protein provides with cells or individual organism can be empirically analyzed.

Where the composition of the present invention is used for the purposes of treatment of diseases, diagnosis and/or treatment such as pharmacokinetic imaging or monitoring *in vivo,* etc., or analysis of the protein *in vivo* for its function, etc., the composition of the present invention may further contain a pharmacologically acceptable vehicle, a diluent or an excipient and can be provided in the form of a pharmaceutical composition suitable for oral or parenteral administration.

Examples of the composition for oral administration include solid or liquid dosage forms, specifically, tablets (including dragees and film-coated tablets), pills, granules, powdery preparations, capsules (including soft capsules), syrup, emulsions, suspensions, etc. Such a composition is prepared by publicly known methods and contains a vehicle, a diluent or excipient conventionally used in the art of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations are prepared by methods publicly known, for example, by dissolving, suspending or emulsifying the fusion protein of the present invention or the expression vector comprising the polynucleotide encoding the fusion protein in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the fusion protein or the expression vector comprising the polynucleotide encoding the fusion protein with conventional bases for suppositories.

Advantageously, the pharmaceutical compositions for parenteral or oral use described above are prepared into pharmaceutical preparations with a unit dose suited to fit a dose of the active ingredients. Such unit dose preparations include, for example, tablets, pills, capsules, injections (ampoules) and suppositories. The amount of the antibody contained is generally about 5 to about 500 mg per dosage unit form; it is preferred that the aforesaid antibody is contained in about 5 to about 100 mg especially in the form of injection, and in about 10 to 250 mg for the other forms. Since the pharmaceutical preparation thus obtained is safe and low toxic, it can be administered orally or parenterally to human or a warm-blooded animal (e.g., mouse, rat, rabbit, sheep, swine, bovine, horse, fowl, cat, dog, monkey, chimpanzee etc.).

A dose of the antibiotic (for example, the tetracycline antibiotic) may vary depending on a target disease, a subject to be administered, a route for administration, etc.; in oral administration, the antibiotic is administered to an adult (as 60 kg body weight) normally in a daily dose of about 0.1 to 500 mg, preferably about 5.0 to 300 mg and more preferably about 5.0 to 200 mg. In parenteral administration, a single dose of the antibiotic varies depending on a subject to be administered, a target disease, etc. but when the tetracycline antibiotic is administered to an adult (as 60 kg body weight) in the form of an injectable preparation, it is advantageous to administer the antibiotic intravenously in a daily dose of about 0.1 to about 300 mg, preferably about 1 to about 200 mg and more preferably about 10 to about 100 mg. For other animal species, the corresponding dose as converted per 60 kg weight can be administered.

The composition of the present invention is used in combination with an antibiotic (e.g., the tetracycline antibiotic). In one embodiment, the composition of the present invention further contains the antibiotic. In another embodiment, the composition of the present invention is administered to a cell or a subject concurrently with the antibiotic, or before or after the antibiotic is administered.

### 4. Kit of the invention

In one aspect, the present invention further provides a kit comprising a fusion protein of the present invention, a kit comprising a polynucleotide encoding the fusion protein of the present invention and a kit comprising an expression vector comprising the polynucleotide encoding the fusion protein of the present invention. The present invention further provides a kit comprising the composition of the present invention described in 3 above.

The kit of the present invention usually further comprises antibiotics (e.g., the tetracycline antibiotic). The kit of the present invention can be used, for example, to prepare and select transformants by introducing the expression vector comprising the polynucleotide encoding the fusion protein of the present invention into a cell *in vitro* or *ex vivo.*

The kit of the present invention may further contain a buffer, a syringe, a vial, etc. required for a desired dosage form for use *in vivo.* Furthermore, the kit of the present invention may contain manufacturer's instructions on how to use and/or precautions, etc.

### 5. Method for controlling the degradation of the protein by the antibiotic of the present invention

The present invention provides a method for controlling the degradation of a protein by an antibiotic capable of intracellular delivery (for example, tetracycline antibiotic). This method comprises either one of the steps (A) and (B) below:
(A) the step of expressing the polynucleotide encoding the fusion protein comprising a variant protein of the protein binding to the antibiotic and a target protein having fused thereto in a cell or *in vivo* in the presence or absence of the antibiotic;
(B) the step of using the fusion protein comprising a variant protein of the protein binding to the antibiotic and a target protein having fused thereto in a cell or *in vivo* in the presence or absence of the antibiotic.

In a preferred embodiment, the aforesaid antibiotic is the tetracycline antibiotic and the protein binding to the antibiotic is a repressor protein of the antibiotic.

One of the preferred embodiments of present invention described above is a method for controlling the degradation of a protein by the tetracycline antibiotic. This method comprises any one of the steps (A) and (B) below:
(A) the step of expressing the polynucleotide encoding the fusion protein comprising a variant TetR protein and a target protein having fused thereto in a cell or *in vivo* in the presence or absence of the tetracycline antibiotic;
(B) the step of using the fusion protein comprising the variant TetR protein and a target protein having fused thereto in a cell or *in vivo* in the presence or absence of the tetracycline antibiotic.

For experimental purposes or for the purposes of diagnosis, prevention or treatment of diseases, for example, the method of the present invention described above can be used to introduce a gene encoding the fusion protein of the present invention into a cell *in vitro* or *ex vivo* to express the gene in the presence or absence of the antibiotic (e.g., the tetracycline antibiotic), or introduce a gene encoding the fusion protein of the present invention into a cell or a tissue or an organ containing such a cell of a subject *in vivo* to express the gene in the presence or absence of the antibiotic (e.g., the tetracycline antibiotic). Introduction of the gene (polynucleotide) encoding the fusion protein of the present invention into a cell can be performed in a manner similar to the method previously described in 2 above.

For experimental purposes or for the purposes of diagnosis, prevention or treatment of diseases, for example, the method of the present invention can further be used to employ the fusion protein of the present invention in a cell *in vitro* or *ex vivo* in the presence or absence of the antibiotic (e.g., the tetracycline antibiotic), or employ the fusion protein of the present invention in a cell or a tissue or an organ containing such a cell of a subject *in vivo* in the presence or absence of the antibiotic (e.g., the tetracycline antibiotic).

In the method of the present invention, the fusion protein described above or the gene encoding the fusion protein described above may be administered or contacted directly to a cell or a tissue, etc. of a subject, and may preferably be formulated together with an appropriate vehicle, diluent or excipient, etc. and introduced into a cell or living body, as described in 3 above. According to the method described above, the antibiotic (e.g., the tetracycline antibiotic) can be administered or provided to a cell or living body before the fusion protein described above or the gene encoding the fusion protein described above is administered or provided to the cell or living body, concurrently with or after the administration. In the method described above, the degradation of the protein can be controlled by regulating (increasing or decreasing) the concentration of the antibiotic (e.g., the tetracycline antibiotic) (in a cell or tissue).

Hereinafter the present invention will be described specifically with reference to EXAMPLES but the scope of the present invention is not deemed to be limited thereto.

### EXAMPLES

### EXAMPLE 1

### Preparation of wild-type TetR-EGFP and variant TetR-EGFP and verification of stability

### 1. Material and method

### A. Preparation of cDNA

All or two of the three mutations that asparagine is substituted for aspartic acid at position 95, serine for leucine at position 101 and aspartic acid for glycine at position 102 were introduced into the tetracycline repressor (TetR), which is an *Escherichia coli* protein binding to the antibiotic tetracycline (Tet). The procedures are as given below.

### (Procedures)

An oligonucleotide encoding an amino acid sequence containing the amino acid substitution to be introduced was synthesized. Using the oligonucleotide, a mutation-containing DNA fragment was prepared by PCR. The mutation described above was introduced by replacing the DNA fragment for wild-type protein-encoding DNA at the corresponding site.

Next, cDNA encoding TetR-EGFP in which genes for the wild-type and mutation-introduced TetR were fused to an enhanced green fluorescence protein (EGFP) was prepared. The procedures are as given below.

### (Procedures)

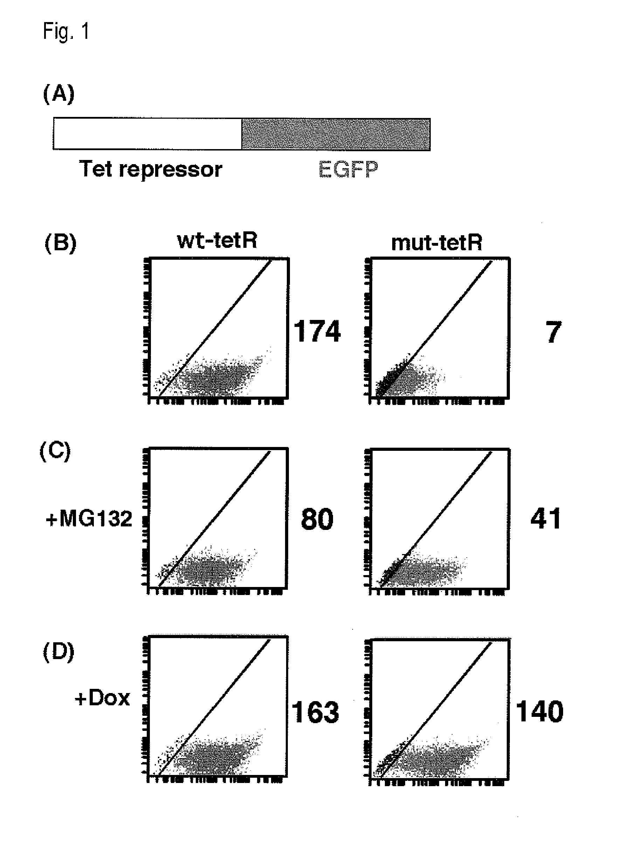

An oligo-DNA having a substituted nucleotide sequence so as to encode other amino acid for the termination codon of TetR gene and a sequence recognized and digested with a restriction enzyme located downstream of the nucleotide sequence was synthesized. Using the oligo-DNA, PCR was carried out to prepare a DNA fragment encoding TetR bearing no termination codon and the DNA fragment was substituted for the corresponding site of DNA encoding the wild-type gene. Next, the DNA fragment was digested with restriction enzymes and ligated with the upstream region of a DNA fragment encoding EGFP to match the translation reading frame for protein synthesis (FIG. 1A).

### B. Preparation of gene expression vector

The cDNA prepared as described above was inserted into gene expression vector "pEB6CAG" developed by the present inventor and collaborators, which can be stably replicated and maintained in human cells. The constructed expression vector DNA was mass-produced from *Escherichia coli* using a commercially available DNA purification kit.

### C. Preparation and selection of transformant

This DNA was transfected into the human cell line HEp-2 using a commercially available lipofection reagent, followed by incubation for 4 days in the presence of 1.5 mg/ml of G418 to select DNA-transfected cells only. The cells were treated with trypsin and then recovered. Fluorescence-positive cell rates and the intensity of fluorescence per cell were analyzed by a flow cytometer (BD, FACSCalibur).

### 2. Results

### A. Measurement of fluorescent intensity by flow cytometer

In FIG. 1, B to D are graphs showing the results obtained when the stability of wild-type TetR-EGFP and variant TetR-EGFP was assessed using a flow cytometer.

In the cells where wild-type TetR-EGFP was expressed, the EGFP-derived fluorescence was observed in more than 90% of the cells, whereas in the variant TetR-EGFP-expressed cells, only a very faint fluorescence was observed in approximately 10-20% of the cells (FIG 1B).

These cells were incubated for 12 hours in the presence of 100 µg/ml of proteasome inhibitor MG132. In the wild-type TetR-EGFP-expressed cells, no significant change in the fluorescent intensity was observed but a marked increase in the fluorescence was observed with the variant TetR-EGFP-expressed cells (FIG. 1C). The results revealed that degradation of the variant TetR-EGFP protein in cells by the proteasome was the cause for the very faint fluorescence observed.

Furthermore, when the cells were incubated for 4 days by adding 1.5 µg/ml of doxycycline thereto, no change was observed with wild-type TetR-EGFP but a more markedly enhanced fluorescence than in the case of adding MG132 was observed (FIG. 1D). In this case, TetR where all of the 3 mutations described above were incorporated was used.

### B. Inverted microscope observation

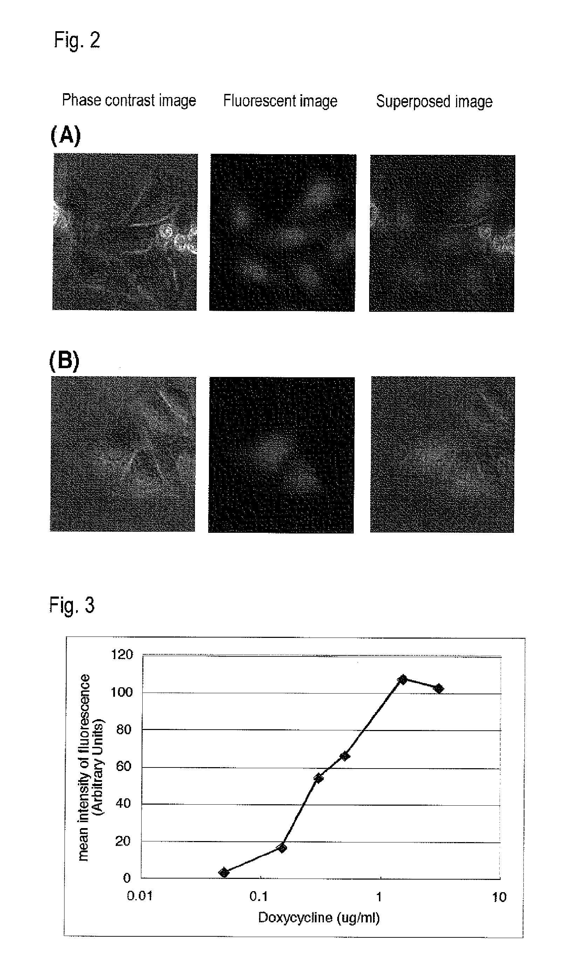

FIG 2A shows photographs of the cells described above, which were observed with an inverted microscope. As shown, the green fluorescence was predominantly observed in the nucleus, although the fluorescence was detected over the whole cells (FIG. 2A). This was assumed to be because the protein was localized predominantly in the nucleus since TetR is a DNA-binding protein and thus binds to genomic DNA in human cells. It is expected that this might cause a problem when it is applied for general purposes. Accordingly, mutation to replace glutamine for arginine at position 28 was introduced to cause loss of the DNA binding ability.

FIG. 2B is photographs showing the results of observation under a fluorescence microscope using the TetR-EGFP variant, to which the R28Q mutation was further introduced. As shown, the localization of the fluorescence in the nucleus was cleared up and the fluorescence was observed uniformly in the cells, resulting in blurring the borders between the nucleus and the cytoplasm. In the present type of mutations, the degree of doxycycline-dependent enhancement of the fluorescence was maintained (FIG. 2B).

### C. Correlation between doxycycline level and fluorescent intensity

To analyze the correlation between the level of doxycycline and fluorescent intensity, the fluorescent intensity was analyzed with a flow cytometer at various concentrations of doxycycline added. The results are shown in FIG 3. As shown, an enhanced fluorescence was observed at concentrations of 0.05 µg/ml or more, showing the maximum fluorescent intensity at 1.5 µg/ml.

### D. Correlation between passage of time and fluorescent intensity after addition of doxycycline

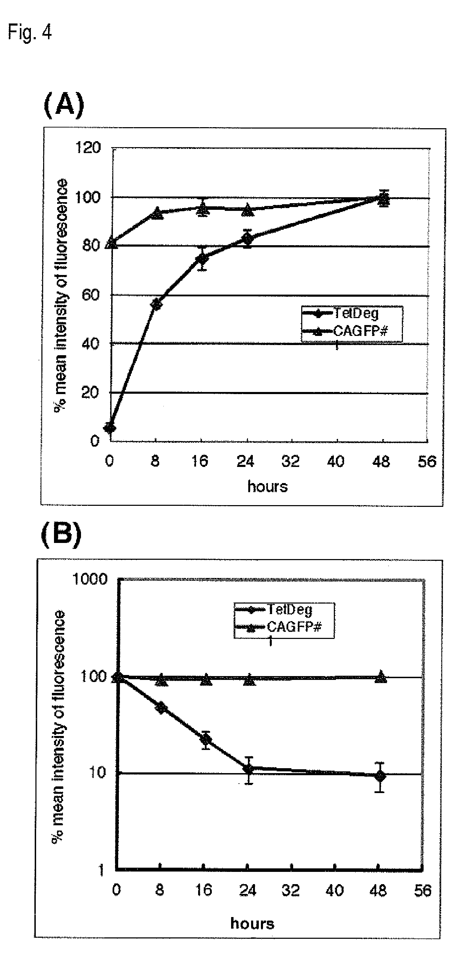

In order to analyze changes with passage of time after addition of doxycycline, the fluorescent intensity was measured every 8 hours after addition of 1.5 µg/ml of doxycycline. The results are shown in FIG. 4A. As shown, when compared to the cells transfected with a vector only expressing EGFP, the cells transfected with a vector expressing the variant TetR-EGFP displayed a sudden increase in the fluorescence for the initial 8 hours and the fluorescence reached almost an equilibrium by 24 hours (FIG. 4A). Conversely, for monitoring changes with the passage of time after removal of doxycycline, the cells were treated with 1.5 µg/ml of doxycycline for 4 days. After the medium was replaced with doxycycline-free MEM medium, the fluorescent intensity was measured every 8 hours. The results are shown in FIG. 4B. As shown, the half-life period was 8 hours and the fluorescence was almost completely quenched after 24 hours (FIG. 4B).

### EXAMPLE 2

### In vivo assessment of the protein degradation control system of the invention

### 1. Assessment in transgenic mouse

In order to analyze if the behavior of doxycycline is detectable as fluorescence in a living animal using the present protein degradation control system, a transgenic mouse was prepared.

cDNA of TetR-EGFP having mutations of R28Q, D95N, L101S and G102D was ligated downstream of the CAG promoter, which is known to readily induce systemic expression of a transfected gene in mouse, and which is followed by the IRES sequence, and cDNA capable of expressing a tandem dimer of fluorescent protein DsRed was placed downstream of the IRES sequence. Using this, it was predicted that degradation of the TetR-EGFP protein would be controlled by the presence or absence of doxycycline such that the green fluorescent intensities fluctuate while the red fluorescence of DsRed would always remain constant. It was therefore expected that it would be possible to monitor changes in the expression intensity of each organ by the red fluorescence, whereas changes in the green fluorescent intensity due to the behavior of doxycycline could be standardized and digitalized by calculating the ratio of green to red fluorescent intensities.

Then, the mass-produced DNAs were injected into fertilized mouse eggs and the eggs were reimplanted into a pseudopregnant mouse. The animal bearing the transgene was selected from the neonatal offspring mice and bred.

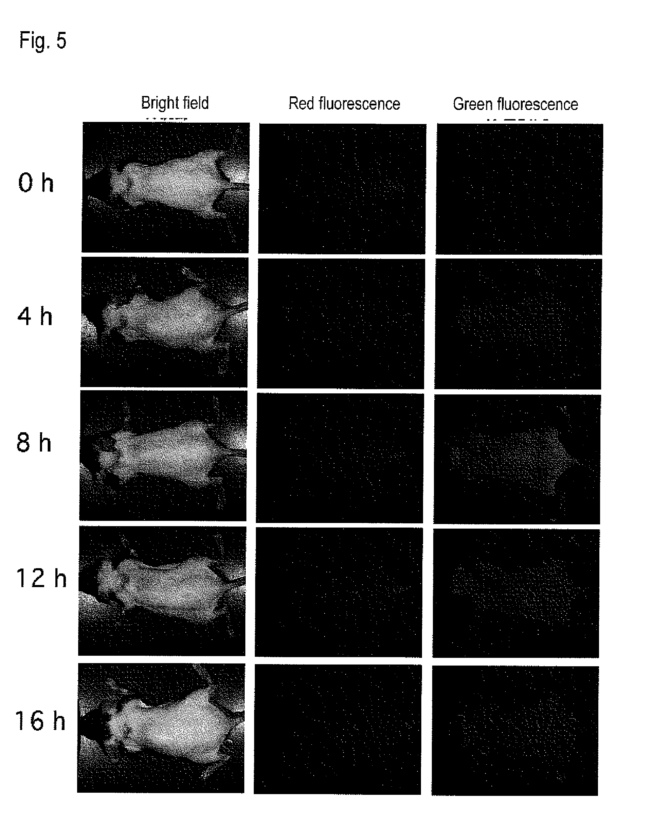

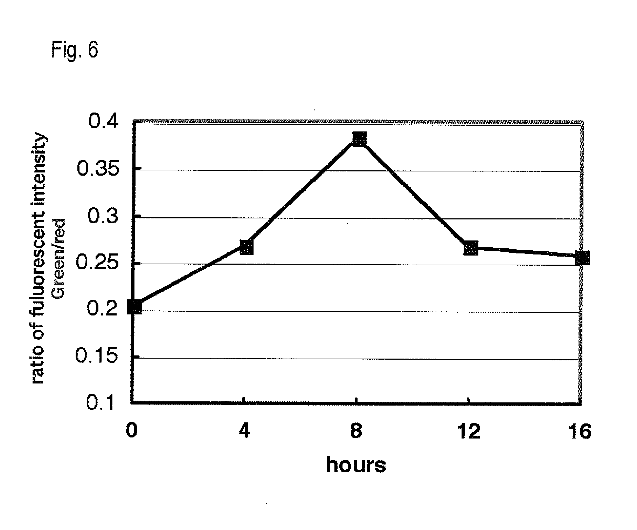

As expected, the red fluorescence was observed in this mouse on a routine basis. Further when doxycycline was intraperitoneally administered, systemic increase of the green fluorescence was found at 8 hours (FIG. 5). The ratio of green to red fluorescent intensities is shown in a graph form, which established that the green fluorescent intensity reached the maximum at 8 hours and then declined (FIG. 6).

### 2. Assessment in zebrafish

In order to confirm if similar degradation control occurs in other animal species, mRNA encoding TetR-EGFP in which mutations R28Q, D95N, L101S and G102D were introduced was synthesized using a kit commercially available and injected into fertilized zebrafish eggs.

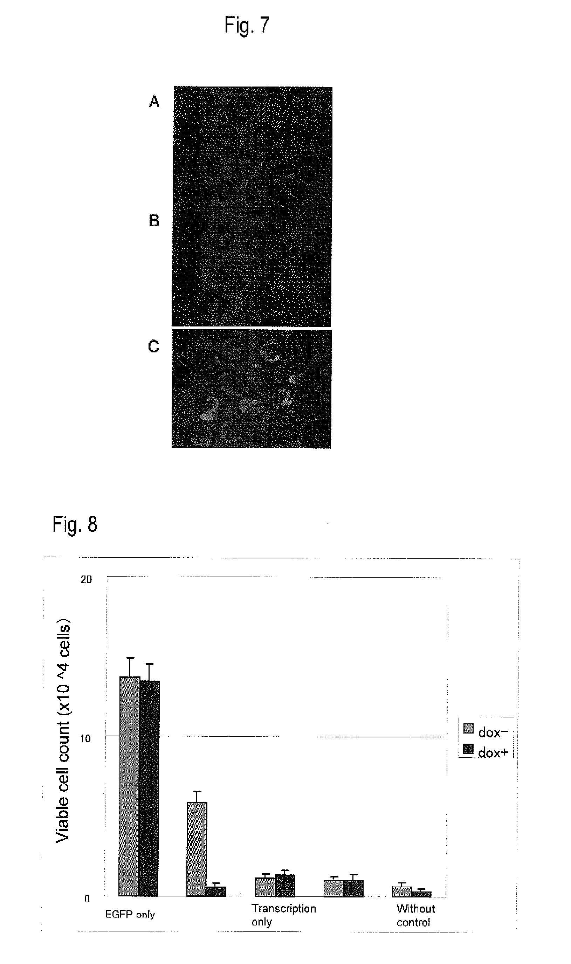

When doxycycline was not added, the green fluorescence was not detected, whereas a weak fluorescence was observed in 40% of the eggs 24 hours after 1.5 µg/ml of doxycycline was added and a very strong fluorescence was observed in 100% of the eggs when 15 µg/ml was added (FIG 7). Thus, it was demonstrated that the degradation control system same as that used for mice could also be used for fish.

### EXAMPLE 3

### Control of gene expression using the fusion protein of the invention

A cDNA encoding TetR-DTA-EGFP produced by fusing a gene encoding diphtheria toxin A (DTA), which is a diphtheria toxoid having a strong toxicity against human cells, with a gene encoding a green fluorescent protein (EGFP) and further fusing resulting DTA-EGFP with a variant TetR was prepared and used in the experiments for controlling transcription of the gene. The procedures are given below.

### (Procedures)

### A. Preparation of TetR-DTA-EGFP

A cDNA fragment encoding DTA was prepared by digesting known pMCl DT-3 vector with restriction enzymes BamHI-DraI. The fragment was inserted into the above variant TetR-EGFP gene, from which the TetR moiety had been removed by digestion with restriction enzymes BglII-SmaI, to prepare the DTA-EGFP-encoding cDNA. In addition, a cDNA fragment encoding DTA was inserted between TetR and EGFP of the TetR-EGFP gene using restriction enzyme BamHI to prepare TetR-DTA-EGFP.

### B. Preparation of gene expression vector

The cDNA prepared as described above was incorporated into homeostatic gene expression vector "pEB6CAG" or vector "pOSTet15", which was developed by the present inventor by applying the method of Japanese Patent Laid-open Publication No. 2003-515314 such that the expression of which could be regulated by doxycycline. The following 4 vectors were thus constructed: 1) "pOSTet15-DTAEGFP" for double control on transcription and protein degradation; 2) "pOSTet15-DTAEGFP" for single control on transcription; 3) "pEB6CAG-TetRDTAEGFP" for single control on protein degradation; and 4) "pEB6CAG-DTAEGFP" for homeostatic expression without any restriction. In addition to these vectors, "pOSTet15TetR-EGFP" free of DTA and showing no cytotoxicity was also constructed for comparative experiment. The constructed expression vector DNA was mass-produced from *Escherichia coli* using a commercially available DNA purification kit.

### C. Preparation and selection of transformant

This DNA was introduced into the human cell line HEp-2 using a commercially available lipofection reagent. Incubation was performed for 4 days in the presence of 1.5 mg/ml G418 and DNA-introduced cells were only selected, in which the viable cells were counted. The fluorescence of EGFP was observed to confirm that the gene was introduced.

### RESULTS

As shown in FIG. 8, when the single control on either transcription or protein degradation was applied, the results are almost the same as those for which no control was applied, and almost all cells were dead even when no doxycycline was added and therefore the expression was supposed to be repressed. This means that single control was not sufficient for repressing the expression, and the cells were extinguished due to high toxicity of DTA even when leakage of the expression was on a low level.

On the other hand, when double control was applied, it was demonstrated that, although the cell count was less than that for cells to which a non-toxic gene was transduced in the absence of doxycycline, the expression of a toxin gene was sufficiently repressed such that a sufficient number of the cells survived. It was further revealed that when doxycycline was then added, the toxin gene was expressed and the cells were extinguished.

The above results show that even when strict control of expression is difficult only by transcription control using doxycycline in accordance with the conventional-art technique, extremely strict gene expression control can be realized by the technique in combination with the protein degradation control according to the present invention.

### INDUSTRIAL APPLICABILITY

According to the present invention, by introducing a fusion gene of the target gene for treatment and the variant TetR gene, for example, the level of the fusion protein as the expression product can be controlled by Tet such that Tet may be administered depending upon the conditions of a patient to perform treatment of the patient while preventing adverse effects. The present invention is thus useful for applications in the medical fields, etc.

Furthermore, according to the present invention, by expressing a variant protein of a protein binding to an antibiotic (e.g., the variant TetR) as a fusion protein with a detectable protein such as a fluorescent protein or a luminescent protein, the amount of the antibiotic (e.g., Tet) in viable cells or individual organisms can be detected by imaging technique because the amounts of fluorescence or luminescence vary depending upon the amount of the antibiotic (e.g., Tet). Accordingly, the present invention is also useful for *in vivo* imaging, etc.

## Claims

1. A fusion protein comprising a variant protein of a protein binding to an antibiotic and a target protein having fused thereto, wherein:
said variant protein is degraded when the variant protein is not bound to said antibiotic but stabilized when the variant protein is bound to said antibiotic in a cell, and,
said fusion protein is degraded when the fusion protein is not bound to said antibiotic but stabilized when the fusion protein is bound to said antibiotic in a cell.

2. The fusion protein according to claim 1, wherein said antibiotic is a tetracycline antibiotic and said protein binding to said antibiotic is a repressor protein of said antibiotic.

3. The fusion protein according to claim 2, comprising a variant TetR protein and a target protein having fused thereto.

4. The fusion protein according to claim 3, wherein said variant TetR protein has an amino acid sequence in which at least one amino acid residue is substituted in the amino acid sequence of wild-type TetR protein.

5. The fusion protein according to claim 4, wherein at least two amino acid residues of aspartic acid at position 95, leucine at position 101 and glycine at 102 are substituted in the amino acid sequence of wild-type TetR protein.

6. The fusion protein according to any one of claims 1 through 5, wherein said target protein is a fluorescent protein or a luminescent protein.

7. The fusion protein according to any one of claims 1 through 5, wherein said target protein is a therapeutic protein.

8. The fusion protein according to any one of claims 1 through 5, wherein said target protein is a protein for subjecting to analysis of its function.

9. The fusion protein according to claim 6, wherein said fluorescent protein or luminescent protein is any one of a green fluorescent protein and a luciferase.

10. A polynucleotide encoding the fusion protein according to any one of claims 1 through 9.

11. An expression vector comprising a polynucleotide encoding the fusion protein according to any one of claims 1 through 9.

12. A host cell or host organism which is transfected by the expression vector according to claim 11.

13. A composition for intracellular or *in vivo* imaging, comprising the fusion protein according to claim 6 or 9.

14. A composition for intracellular or *in vivo* imaging, comprising an expression vector comprising a polynucleotide encoding the fusion protein according to claim 6 or 9.

15. A therapeutic composition, comprising the fusion protein according to claim 7.

16. A therapeutic composition, comprising an expression vector comprising a polynucleotide encoding the fusion protein according to claim 7.

17. A composition for analysis of protein functions, comprising the fusion protein according to claim 8.

18. A composition for analysis of protein functions, comprising an expression vector comprising a polynucleotide encoding the fusion protein according to claim 8.

19. The composition according to any one of claims 13 through 18, which is used in combination with a tetracycline antibiotic.

20. The composition according to claim 19, wherein said tetracycline antibiotic is tetracycline or its derivative selected from doxycycline, oxytetracycline, chlorotetracycline, or anhydrotetracycline.

21. A kit comprising the fusion protein according to any one of claims 1 through 9, the polynucleotide according to claim 10, the expression vector according to claim 11, or the composition according to any one of claims 13 through 20.

22. A method for controlling the degradation of a protein by an antibiotic capable of being introduced into a cell, comprising any one of the steps (A) and (B):
(A) the step of expressing a polynucleotide encoding a fusion protein comprising a variant protein of a protein binding to said antibiotic and a target protein having fused thereto in a cell or *in vivo* in the presence or absence of said antibiotic, and,
(B) the step of using a fusion protein comprising a variant protein of a protein binding to said antibiotic and a target protein having fused thereto in a cell or *in vivo* in the presence or absence of said antibiotic, wherein:
said variant protein is degraded when the variant protein is not bound to said antibiotic but stabilized when the variant protein is bound to said antibiotic in a cell, and,
said fusion protein is degraded when the fusion protein is not bound to said antibiotic but stabilized when the fusion protein is bound to said antibiotic in a cell.

23. The method according to claim 22, wherein said antibiotic is a tetracycline antibiotic and said protein binding to the antibiotic is a repressor protein of said antibiotic.

24. A method for controlling the degradation of a protein by a tetracycline antibiotic, comprising any one of the steps (A) and (B):
(A) the step of expressing a polynucleotide encoding a fusion protein comprising a variant TetR protein and a target protein having fused thereto in a cell or *in vivo* in the presence or absence of the tetracycline antibiotic, and,
(B) the step of using a fusion protein comprising a variant TetR protein and a target protein having fused thereto in a cell or *in viva* in the presence or absence of the tetracycline antibiotic.

25. The method according to claim 23 or 24, wherein the degradation of said target protein is controlled by regulating the concentration of said tetracycline antibiotic.

26. The method according to claim 24 or 25, wherein said variant TetR protein has an amino acid sequence in which at least two amino acid residues of aspartic acid at position 95, leucine at position 101 and glycine at 102 are substituted in the amino acid sequence of wild-type TetR protein.

27. The method according to any one of claims 22 through 26, wherein said target protein is a therapeutic protein.

28. The method according to any one of claims 22 through 26, wherein said target protein is a protein for subjecting to analysis of its function.

29. The method according to any one of claims 22 through 26, wherein said target protein is a fluorescent protein or a luminescent protein.

30. The method according to any one of claims 23 through 29, wherein said tetracycline antibiotic is tetracycline or its derivative selected from doxycycline, oxytetracycline, chlorotetracycline, or anhydrotetracycline.


SEQUENCE LISTING

<110>  JST

<120>  Method of controlling degradation of protein by tetracycline
       antibiotic

<130>  G08-0001

<140>  PCT/JP2006/318673
<141>  2006-09-14

<150>  JP 2005-269074
<151>  2005-09-15

<160>  2

<170>  PatentIn version 3.3

<210>  1
<211>  624
<212>  DNA
<213>  Serratia marcescens

<400> 1

atgtctagat tagataaaag taaagtgatt aacagcgcat tagagctgct taatgaggtc 60

ggaatcgaag gtttaacaac ccgtaaactc gcccagaagc taggtgtaga gcagcctaca 120

ttgtattggc atgtaaaaaa taagcgggct ttgctcgacg ccttagccat tgagatgtta 180

gataggcacc atactcactt ttgcccttta gaaggggaaa gctggcaaga ttttttacgt 240

aataacgcta aaagttttag atgtgcttta ctaagtcatc gcgatggagc aaaagtacat 300

ttaggtacac ggcctacaga aaaacagtat gaaactctcg aaaatcaatt agccttttta 360

tgccaacaag gtttttcact agagaatgca ttatatgcac tcagcgctgt ggggcatttt 420

actttaggtt gcgtattgga agatcaagag catcaagtcg ctaaagaaga aagggaaaca 480

cctactactg atagtatgcc gccattatta cgacaagcta tcgaattatt tgatcaccaa 540

ggtgcagagc cagccttctt attcggcctt gaattgatca tatgcggatt agaaaaacaa 600

cttaaatgtg aaagtgggtc ttaa 624

<210> 2

<211> 207

<212> PRT

<213> Serratia marcescens

<400> 2

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg

65                  70                  75                  80

Asn Asn Ala Lys Ser Phe Arg Cys Ala Leu Leu Ser His Arg Asp Gly

                85                  90                  95

Ala Lys Val His Leu Gly Thr Arg Pro Thr Glu Lys Gln Tyr Glu Thr

            100                 105                 110

Leu Glu Asn Gln Leu Ala Phe Leu Cys Gln Gln Gly Phe Ser Leu Glu

        115                 120                 125

Asn Ala Leu Tyr Ala Leu Ser Ala Val Gly His Phe Thr Leu Gly Cys

    130                 135                 140

Val Leu Glu Asp Gln Glu His Gln Val Ala Lys Glu Glu Arg Glu Thr

145                 150                 155                 160

Pro Thr Thr Asp Ser Met Pro Pro Leu Leu Arg Gln Ala Ile Glu Leu

                165                 170                 175

Phe Asp His Gln Gly Ala Glu Pro Ala Phe Leu Phe Gly Leu Glu Leu

            180                 185                 190

Ile Ile Cys Gly Leu Glu Lys Gln Leu Lys Cys Glu Ser Gly Ser

        195                 200                 205